# DEMANDE DE BREVET EUROPEEN

(11) **EP 3 835 406 A1**
(43) Date de publication de la demande: **16.06.2021**
(21) Numéro de dépôt: 20210396.6
(22) Date de dépôt: 27.11.2020
(51) Int. Cl.: C12M 1/107, C10L 3/10, C12M 1/00

(54) **DIGESTEUR COMPRENANT UNE PAROI INTERNE PRÉSENTANT DES EXPANSIONS ET/OU DES CREUX**

(30) Priorité: 11.12.2019 FR 1914169
(71) Demandeur: L'AIR LIQUIDE, SOCIETE ANONYME POUR L'ETUDE ET L'EXPLOITATION DES PROCEDES GEORGES CLAUDE, 75007 Paris (FR)
(72) Inventeur: BERTRANDIAS, Aude, 78350 Les Loges-En-Josas (FR); FRIMAT, David, 78350 Les Loges-En-Josas (FR); TRUEBA, Antonio, 78350 Les Loges-En-Josas (FR); SEIWERT, Jacopo, 78350 Les Loges-En-Josas (FR); OLLIER, Jérémy, 38360 Sassenage (FR); VALENTIN, Solène, 38360 Sassenage (FR)
(74) Mandataire: Air Liquide

(57) **Abrégé**

Installation de production de biogaz au moins partiellement désulfuré comprenant un digesteur et/ou un post-digesteur de biomasse, le digesteur et/ou le post-digesteur comprenant :
- Une enceinte comprenant la biomasse et le ciel gazeux, et
- Un moyen d'introduction d'un gaz d'oxydation,
caractérisée en ce que la portion de la paroi interne de l'enceinte située au niveau du ciel gazeux présente des expansions et/ou des creux.

## Description

La présente invention est relative à une installation et un procédé pour la production de biogaz au moins partiellement désulfuré.

Le biogaz est le gaz produit lors de la dégradation de matières organiques en l'absence d'oxygène (fermentation anaérobie) encore appelée méthanisation. Il peut s'agir d'une dégradation naturelle - on l'observe ainsi dans les marais ou les décharges d'ordures ménagères - mais la production de biogaz peut aussi résulter de la méthanisation de déchets dans un réacteur dédié, et dont les conditions sont contrôlées, appelé méthaniseur ou digesteur, puis dans un post-digesteur, similaire au digesteur et permettant de pousser plus loin la réaction de méthanisation.

On appellera biomasse tout groupement de matières organiques pouvant se transformer en énergie à travers ce processus de méthanisation, par exemple les boues de station d'épuration, fumiers/lisiers, résidus agricoles, déchets alimentaires...

Le digesteur, c'est-à-dire le réacteur dédié à la méthanisation de la biomasse, est une cuve fermée, chauffée ou non (opération à une température fixée, entre la température ambiante et 55°C) et dont le contenu constitué de la biomasse est brassé, en continu ou séquentiel. Les conditions dans le digesteur sont anaérobies et le biogaz généré se retrouve dans l'espace de tête du digesteur (ciel gazeux), où il est prélevé. Les post-digesteurs sont similaires aux digesteurs.

De par ses constituants principaux - méthane et dioxyde de carbone - le biogaz est un puissant gaz à effet de serre ; il constitue aussi, parallèlement, une source d'énergie renouvelable appréciable dans un contexte de raréfaction des énergies fossiles.

Le biogaz contient majoritairement du méthane (CH₄) et du dioxyde de carbone (CO₂) dans des proportions variables en fonction du mode d'obtention et du substrat mais peut également contenir, en moindres proportions de l'eau, de l'azote, de l'hydrogène sulfuré (H₂S), de l'oxygène, ainsi que des composés organiques autres, à l'état de traces, dont le H2S, entre 10 et 50,000 ppmv.

Selon les matières organiques dégradées et les techniques utilisées, les proportions des composants diffèrent, mais en moyenne le biogaz comporte, sur gaz sec, de 30 à 75% de méthane, de 15 à 60% de CO₂, de 0 à 15% d'azote, de 0 à 5% d'oxygène et des composés traces.

Le biogaz est valorisé de différentes manières. Il peut, après un traitement léger, être valorisé à proximité du site de production pour fournir de la chaleur, de l'électricité ou un mélange des deux (la cogénération); la teneur importante en dioxyde de carbone réduit son pouvoir calorifique, augmente les coûts de compression et de transport et limite l'intérêt économique de sa valorisation à cette utilisation de proximité.

Une purification plus poussée du biogaz permet sa plus large utilisation, en particulier, une purification poussée du biogaz permet d'obtenir un biogaz épuré aux spécifications du gaz naturel et qui pourra lui être substitué ; le biogaz ainsi purifié est le « biométhane ». Le biométhane complète ainsi les ressources de gaz naturel avec une partie renouvelable produite au cœur des territoires; il est utilisable pour exactement les mêmes usages que le gaz naturel d'origine fossile. Il peut alimenter un réseau de gaz naturel, une station de remplissage pour véhicules, il peut aussi être liquéfié pour être stocké sous forme de gaz naturel liquide (bioGNL)...

Selon la composition de la biomasse, le biogaz produit lors de la digestion contient du sulfure d'hydrogène (H₂S) dans des teneurs comprises entre 50 et 50 000 ppm.

Quelle que soit la destination finale de valorisation du biogaz, il s'avère indispensable d'éliminer le sulfure d'hydrogène qui est une impureté toxique et corrosive. De plus, si l'utilisation du biogaz consiste à l'épurer pour injecter du biométhane dans le réseau de gaz naturel, des spécifications strictes limitent la quantité de H₂S autorisée.

Plusieurs méthodes d'élimination du H₂S existent et sont plus ou moins répandues (lits de charbon actif, ajout de composés de fer, absorption physique ou chimique, lavages à l'eau, biofiltres...). L'élimination se fait principalement par adsorption sur lit de charbon actif, à l'extérieur du digesteur. Dans un nombre croissant de digesteurs, l'abattement de l'H2S se fait aussi en partie par injection d'air/air enrichi/O₂ dans le ciel gazeux du digesteur, ce qui constitue une solution in situ. Avec une injection dans le ciel gazeux à faible dose, du soufre solide est formé à partir du H₂S et O₂ (eq. (1)), réalisée par des bactéries sulfo-oxydantes e.g. Thiobacillus. A haute dose d'O₂ injecté, le milieu est acidifié (eq. (2)). La réaction (1) est donc ciblée.

H₂S + 0.5 O₂ → S + H₂O (1)

H₂S + 2 O₂ → SO₄²⁻ + 2 H + (2)

Les quantités d'injection d'O₂ nécessaires en pratiques sont différentes de celles attendues par la stoechiométrie de l'éq. (1): des doses de 0.3 - 3% d'O₂ par rapport au biogaz généré sont le plus souvent recommandées, avec des doses jusqu'à 12% qui sont parfois évoquées. Aujourd'hui, l'injection d'air/air enrichi/O₂ in situ n'est pas optimisée et les lits de charbon actif doivent donc être maintenus pour éliminer la totalité du H₂S.

Partant de là, un problème qui se pose est de fournir une installation améliorée favorisant l'élimination plus poussée de l'H₂S.

Une solution de la présente invention est une installation de production de biogaz au moins partiellement désulfuré comprenant un digesteur et/ou un post-digesteur de biomasse, le digesteur et/ou le post-digesteur comprenant :
- Une enceinte comprenant la biomasse et le ciel gazeux, et
- Un moyen d'introduction d'un gaz d'oxydation,
caractérisée en ce que la portion de la paroi interne de l'enceinte située au niveau du ciel gazeux présente des expansions et/ou des creux.

De préférence, les expansions et les creux permettent de multiplier par au moins 1,5, de préférence au moins 2, plus préférentiellement au moins 3 fois la surface « standard » de la portion de paroi interne de l'enceinte située au niveau du ciel gazeux. Par surface « standard » on entend une surface lisse, c'est-à-dire sans expansion et/ou creux.

Par « ciel gazeux » on entend l'espace dans le digesteur ou post-digesteur contenant du gaz (par opposition à l'espace qui contient le liquide).

Les réactions entre l'O2 et l'H2S se produisent sur une surface (pas dans la phase gazeuse ou liquide du digesteur). Les micro-organismes nécessaires à la réaction (bactéries sulfo-oxydantes telles que Thiobacillus) sont dans la phase liquide et les réactifs sont en phase gazeuse. Une surface hydrophile est nécessaire pour mettre en contact la phase liquide, les micro-organismes et la phase gazeuse, les réactifs. Ces surfaces sont principalement les parois internes dans le ciel gazeux, l'interface gaz/liquide du digesteur ou post-digesteur. Si les surfaces disponibles sont insuffisantes, les réactions d'abattement d'H2S ne se produisent pas suffisamment voire pas du tout.

Autrement dit la solution selon l'invention, en augmentant la surface de la paroi interne de l'enceinte permet d'augmenter la taille du support réactionnel et favorise ainsi l'élimination plus poussée du sulfure d'hydrogène.

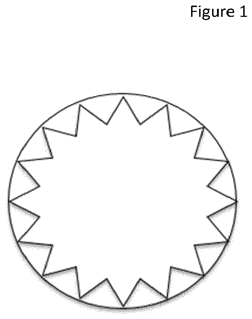

Selon le cas l'installation selon l'invention peut présenter une ou plusieurs des caractéristiques ci-dessous :
- les expansions font saillies vers l'intérieur de l'enceinte,
- les expansions font saillie radialement,
- les expansions et/ou les creux sont successifs le long du périmètre de l'enceinte,
- la portion de paroi interne de l'enceinte située au niveau du ciel gazeux présente une forme de zigzag ; Cette forme zig-zag est représentée à la figure 1 [Fig. 1] qui est une coupe schématique horizontale de l'enceinte du digesteur ou post-digesteur. Notons que la coupe est faite dans la portion de la paroi interne de l'enceinte située au niveau du ciel gazeux.
- la paroi interne est composée d'un matériau résistant à une atmosphère humide et corrosive ; Nous pouvons citer à titre d'exemple certains aciers inox, PEEK, PTFE,
- la paroi interne est divisée en une première paroi et une deuxième paroi, les deux parois étant accolées, et la deuxième paroi étant en regard de l'intérieur de l'enceinte et présentant une surface supérieure à la surface de la première paroi.

La présente invention a également pour objet un procédé de production de biogaz au moins partiellement désulfuré mettant en œuvre une installation selon l'invention, comprenant :
- L'injection de biomasse dans le digesteur ;
- L'injection d'un gaz d'oxydation en tête du digesteur ; et
- Le brassage de la biomasse.

Notons que le gaz d'oxydation pourra être de l'oxygène ou de l'air ou de l'air enrichi. Par air enrichi on entend de l'air présentant une teneur en oxygène plus élevée que la teneur en oxygène habituellement comprise dans l'air.

A l'intérieur du digesteur, lorsque le sulfure d'hydrogène réagit avec l'oxygène, le soufre se fixe sur la paroi interne de l'enceinte du digesteur. Au bout d'un certain temps, le soufre solide généré tombe dans le digestat et est évacué avec celui-ci.La solution selon l'invention permet d'obtenir un flux de biogaz comprenant moins de 200 ppm de sulfure d'hydrogène. L'invention permet de réduire le coût d'épuration du sulfure d'hydrogène du biogaz de façon efficace, en augmentant la réactivité de l'oxygène déjà injecté avec les produits soufrés grâce à la création d'une surface réactionnelle additionnelle sur les parois internes du digesteur au niveau du ciel gazeux, sans besoin complexe en ingénierie.

## Revendications

1. Installation de production de biogaz au moins partiellement désulfuré comprenant un digesteur et/ou un post-digesteur de biomasse, le digesteur et/ou le post-digesteur comprenant :
Une enceinte comprenant la biomasse et le ciel gazeux, et
Un moyen d'introduction d'un gaz d'oxydation,
**caractérisée en ce que** la portion de la paroi interne de l'enceinte située au niveau du ciel gazeux présente des expansions et/ou des creux.

2. Installation selon la revendication 1, **caractérisée en ce que** les expansions font saillies vers l'intérieur de l'enceinte.

3. Installation selon la revendication 2, **caractérisée en ce que** les expansions font saillie radialement.

4. Installation selon l'une des revendications 1 ou 2, **caractérisée en ce que** les expansions et/ou les creux sont successifs le long du périmètre de l'enceinte.

5. Installation selon l'une des revendications 1 à 4, **caractérisée en ce que** la portion de paroi interne de l'enceinte située au niveau du ciel gazeux présente une forme de zigzag.

6. Installation selon l'une des revendications 1 à 4, **caractérisée en ce que** la paroi interne est composée d'un matériau résistant à une atmosphère humide et corrosive.

7. Installation selon l'une des revendications 1 à 6, **caractérisée en ce que** la paroi interne est divisée en une première paroi et une deuxième paroi, les deux parois étant accolées, et la deuxième paroi étant en regard de l'intérieur de l'enceinte et présentant une surface supérieure à la surface de la première paroi.

8. Procédé de production de biogaz désulfuré mettant en œuvre une installation selon l'une des revendications 1 à 7, comprenant :
- L'injection de biomasse dans le digesteur ;
- L'injection d'un gaz d'oxydation en tête du digesteur ; et
- Le brassage de la biomasse.

9. Procédé selon la revendication 8, **caractérisé en ce que** le brassage de la biomasse est réalisé de manière à favoriser un transfert du gaz d'oxydation vers la paroi interne de l'enceinte.

10. Procédé selon l'une des revendications 8 ou 9, **caractérisé en ce que** le gaz d'oxydation est de l'oxygène ou de l'air ou de l'air enrichi.
